# EUROPEAN PATENT SPECIFICATION

(11) **EP 0 672 146 B1**
(45) Date of publication and mention of the grant of the patent: **23.03.2005**
(21) Application number: 93923599.0
(22) Date of filing: 22.10.1993
(51) Int. Cl.: C12N 15/29, C12N 15/82, C07K 7/08, C12N 1/21, A01H 5/00, A01N 65/00

(54) **BIOCIDAL CHITIN BINDING PROTEINS**
BIOZIDE CHITIN BINDENDE PROTEINE
PROTEINES BIOCIDES FIXANT LA CHITINE

(30) Priority: 12.11.1992 GB 9223708; 23.02.1993 GB 9303564
(43) Date of publication of application: 20.09.1995
(73) Proprietor: Syngenta Limited, Guildford Surrey GU2 7YH (GB)
(72) Inventor: BROEKAERT, Willem Frans, B-1700 Dilbeek (BE); CAMMUE, Bruno Philippe Angelo, B-1652 Alsemberg (BE); OSBORN, Rupert William, Twickenham, Middlesex TW2 6SW (GB); REES, Sarah Bronwen, Bracknell, Berkshire RG12 3XX (GB)
(74) Representative: Thomas, Mair Denise
(86) International application number: PCT/GB1993/002179
(87) International publication number: WO 1994/011511

(56) References cited:
- WO-A-92/15691
- WO-A-92/21699
- BIOCHEMISTRY vol. 31, no. 17 , 5 May 1992 , EASTON, PA US pages 4308 - 4314 BROEKAERT, W.F., ET AL. 'Antimicrobial peptides from Amaranthus caudatas seeds with sequence homology to the cysteine/glycine-rich domain of chitin-binding proteins' cited in the application
- BIOLOGICAL ABSTRACTS Abstract no. BR39:62656 see abstract & 90TH ANNUAL MEETING OF THE AMERICAN SOCIETY FOR MICROBIOLOGY 1990. ABSTR. ANNU. MEET. AM. SOC. MICROBIOL. vol. 90 , 1990 page 384 SIDDIQUI, R., ET AL. 'Antimicrobial and immunomodulating proerties of Delphinium -denudatum extracts'
- BIOLOGICAL ABSTRACTS, xol. 63 1977, Philadelphia, PA, US; abstract no. 28390, SABER, M.S.M. 'Antimicrobial substances in certain members of Solanaceae: IV. Detection of active principles in pepper plant' & ZENTRALBL. BAKTERIOL. PARASITENKD. INFEKTIONSKR. HYG. ZWEITE-NATURWISS. ABT. ALLG. LANDWIRTSCH. TECH. MIKRBIOL. vol. 131, no. 2 , 1976 pages 110 - 112
- BIOLOGICAL ABSTRACTS, xol. 65 1978, Philadelphia, PA, US; abstract no. 73020, MOLOT, P.M., ET AL. 'Resistance of sweet pepper (Capsicum annum) to Phytophthora capsici Leon: III. Contamination induced antifungal activity in extracts from susceptible or resistant stems' & ANN. PHYTOPATHOL. vol. 8, no. 4 , 1976 pages 399 - 410

## Description

This invention relates to biocidal proteins, processes for their manufacture and use, and DNA sequences encoding them. In particular it relates to a class of antimicrobial proteins including a protein capable of being isolated from seeds of Capsicum and a protein capable of being isolated from seeds of Briza.

In this context, antimicrobial proteins are defined as proteins possessing at least one of the following activities: antifungal activity (which may include anti-yeast activity); antibacterial activity. Activity includes a range of antagonistic effects such as partial inhibition or death. Such proteins may be oligomeric or may be single peptide subunits.

The genus Capsicum comprises fifty species and includes many important vegetable species which are grown throughout the world (for example, green and red peppers, chillies, paprika and cayenne pepper). As well as these widely cultivated examples, Capsicum also includes a number of species which are grown for their colourful but inedible fruits.

The genus Briza comprises many ornamental grasses and belongs to the Gramineae family. The genus is closely related to grass species found in high-grade temperate pasture, such as rye grass.

Plants produce a wide array of antifungal compounds to combat potential invaders and over the last ten years it has become clear that proteins with antifungal activity form an important part of these defences. Several classes of proteins have been described including thionins, beta-1,3-glucanases, ribosome-inactivating proteins and chitinases. This last group of enzymes falls into a wider class hereafter referred to as the Chitin-binding Plant Proteins. Basic chitinases have been isolated from bean (Boller et al, 1983, Planta, 157:22-31), wheat (Molano et al, 1979, J Biol Chem, 254:4901-4907), tobacco (Shinshi et al, 1987, Proc Nat Acad Sci USA,84:89-93) and other plants. The other known Chitin-binding Plant Proteins have no defined catalytic activity and have thus been described solely on their lectin activity. These include chitin-binding lectins from wheat (Rice and Etzler, 1974, Biochem Biophys Res Comm, 59:414-419), barley (Peumans et al, 1982, Biochem J, 203:239-143), rice (Tsuda, 1979, J Biochem, 86:1451-1461) and stinging nettle (Peumans et al, 1983, FEBS Lett, 177:99-103) plus a small protein from the latex of the rubber tree, called hevein (Van Parijs et al, 1991, Planta,183:258-264).

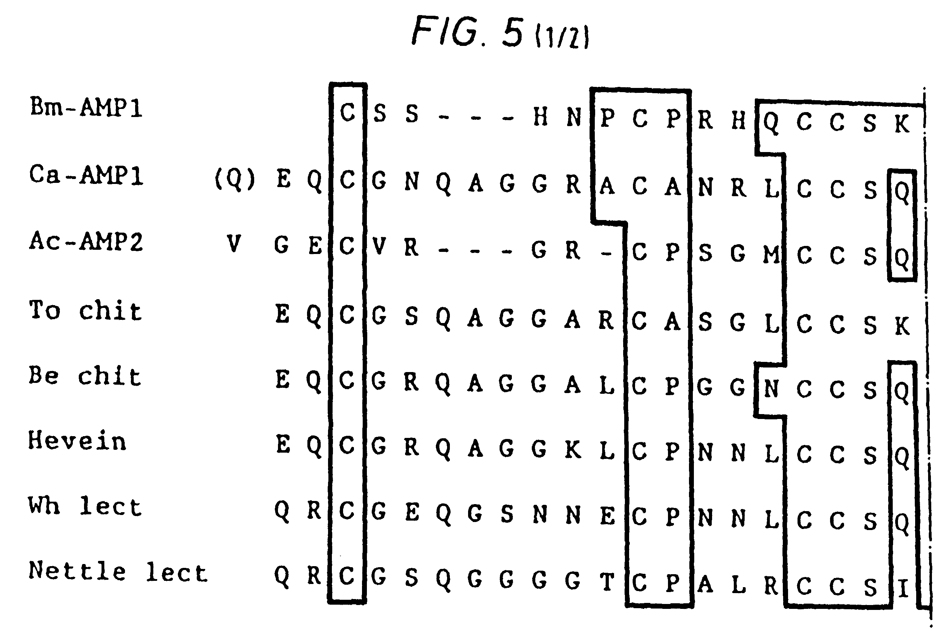

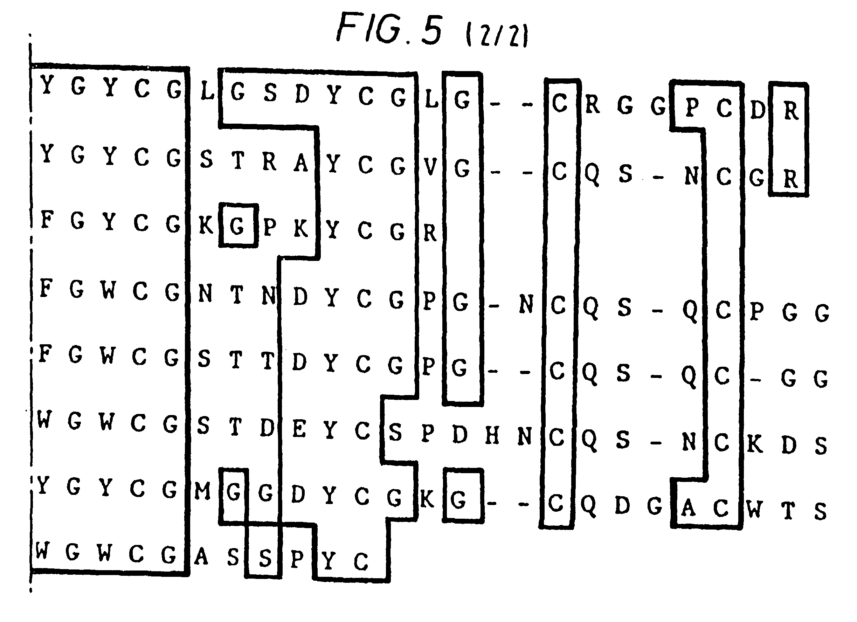

Thus the Chitin-binding Plant Proteins (as herein defined) are a protein group consisting of chitinases, chitin-binding lectins and hevein. All these proteins contain a conserved cysteine/glycine rich domain (for a review see Raikhel and Broekaert, 1991, in Control of plant gene expression, Verma DP (ed), Telford Press). This common region may confer the chitin-binding activity. The domain is 40-43 amino acids in length and is either repeated twice (nettle lectin), four-fold (in wheat, barley and rice lectins) or fused to an unrelated domain (in basic chitinases and prohevein). Hevein itself is 43 amino acids in length and comprises essentially just this conserved domain (Broekaert et al, 1990, Proc Nat Acad Sci USA, 87:7633-7637). A cDNA clone (HEV1) encoding hevein has been isolated (Raikhel and Broekaert, US Patent Number 5187262, published 16 February 1993). Figure 5 shows the common cysteine/glycine-rich domain found in the following Chitin-binding Plant Proteins: tobacco chitinase, bean chitinase, hevein, wheat lectin, nettle lectin. Sequence identities and conserved changes are boxed (conserved changes are considered as substitutions within the amino acid homology groups FWY, MILV, RKH, ED, NQ, ST and PAG; gaps introduced for maximum alignment are represented by dashes). The central region of nine amino acid residues is a particularly well conserved feature of the domain and has the sequence: 
cysteine-cysteine-(serine or threonine)-
1          2          3
(any residue)-(tryptophan, tyrosine or phenylalanine)
 4              5
-glycine-(tryptophan, tyrosine or phenylalanine)-
 6        7
-cysteine-glycine.
 8        9
 Around this core region, the central cysteine motif of the cysteine/glycine rich domain is also absolutely conserved and has the sequence: cysteine-(four amino acids)-cysteine-cysteine-(five amino acids)-cysteine-(six amino acids)-cysteine.

The Chitin-binding Plant Proteins have been found to affect the growth of certain organisms that contain chitin (fungi or insects). However there are differences in the specificity of the proteins. For example, the wheat/barley/rice-type lectins are toxic to weevils, but are inactive to fungi in vitro (Murdock et al, 1990, Phytochem, 29: 85-89). On the other hand, hevein and the chitinases have been found to be inhibitory to the growth of certain pathogenic fungi in vitro (Van Parijs et al, 1991 , Planta, 183: 258-264 ; Broekaert et al, 1988, Physiol Mol Plant Path, 33: 319-331). The HEV1 protein can be used to inhibit the growth of fungi (Raikhel and Broekaert, US Patent Number 5187262, published 16 February 1993). Nettle lectin has also been shown to exert antifungal activity in vitro and at a level 2- to 5-fold greater than hevein (Broekaert et al, 1989, Science, 245: 1100-1102). The potential usefulness of these proteins to engineer resistance in plants has been described (for example, Pioneer Hi-Bred's European Patent Application 502718).

We have previously described two proteins isolated from Amaranthus (the Ac-AMPs) with broad spectrum antifungal activity (Broekaert et al,1992, Biochemistry, 31:4308-4314; International Patent Publication Number WO92/21699) which essentially comprise the cysteine/glycine domain identified in chitin-binding lectins.

The present invention, describes an antimicrobial protein having an amino acid sequence containing the common cysteine/glycine domain of Chitin-binding Plant Proteins and having one or more of the following properties:
substantially better activity against plant pathogenic fungi than that of the Chitin-binding Plant Proteins;
a higher ratio of basic amino acids to acidic amino acids than the Chitin-binding Plant Proteins;
activity against plant pathogenic fungi resulting in hyphal branching.

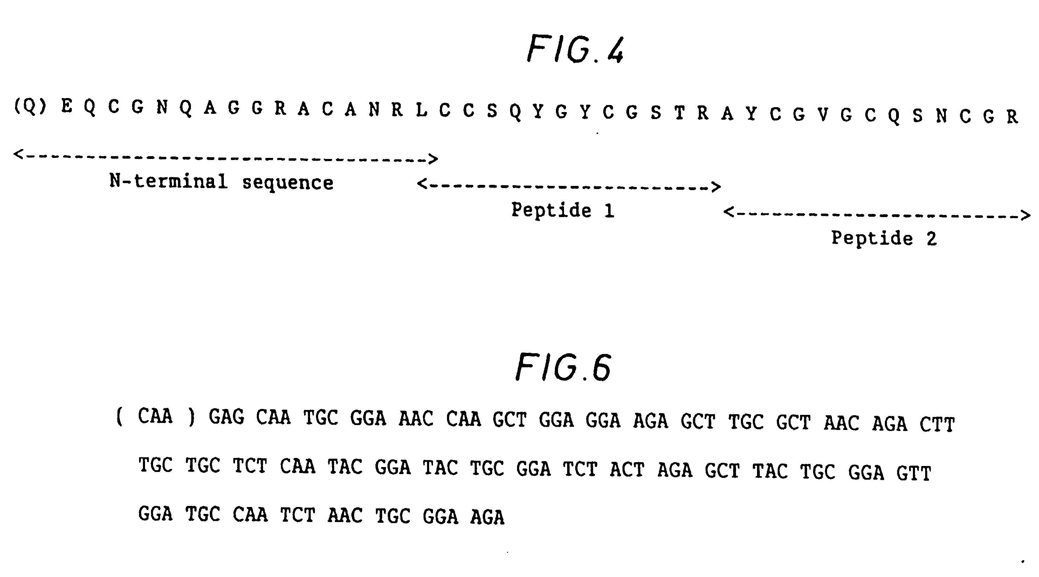

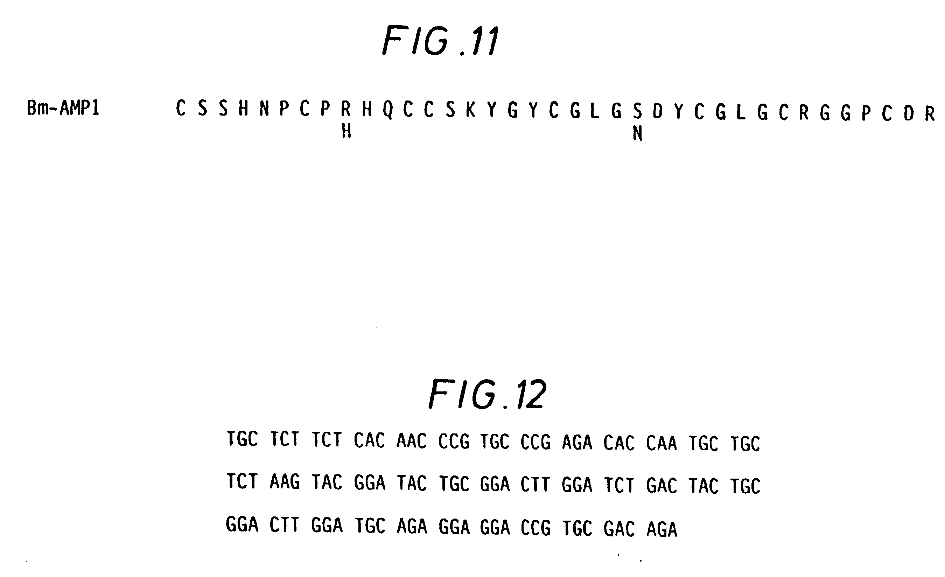

In particular there is provided an antimicrobial protein comprising the amino acid sequence shown in Figure 4 or Figure 11. These proteins are capable of being isolated from seeds of Capsicum species or Briza species. Such antimicrobial proteins may also be isolated from the seeds of both related and unrelated species (including Catapodium, Baptisia, Microsensis, Delphinium), or may be produced or synthesised by any suitable method.

We have purified a new antimicrobial protein from seeds of Capsicum annuum, hereafter called Ca-AMP1 (Capsicum annuum anti-microbial protein 1). The protein shares the common cysteine/glycine domain of Chitin-binding Plant Proteins, but is unique as it possesses very potent and broad spectrum antifungal activity which is at least an order of magnitude greater than hevein or nettle lectin. So despite the conserved nature of these protein sequences (for example, the amino acid sequence for Ca-AMP1 is 65% identical to hevein), the Capsicum protein is markedly improved in the potency and spectrum of its antifungal activity. Indeed, it is remarkable that Ca-AMP1 and hevein are so similar in size and amino acid sequence, but differ so dramatically in their levels and spectrum of activity.

We have also purified a new antimicrobial protein from seeds of Briza maxima, hereafter called Bm-AMP1 (Briza maxima anti-microbial protein 1). The protein shares the common cysteine/glycine domain of Chitin-binding Plant Proteins, but is unique as it possesses very potent and broad spectrum antifungal activity. So despite the conserved nature of these protein sequences, the Briza protein is markedly improved in the potency and spectrum of its antifungal activity. The amino acid sequence for Bm-AMP1 is 45% identical to Ca-AMP1 but only 35% to hevein.

The antifungal activity of Ca-AMP1 and of Bm-AMP1 is similar to that of the Amaranthus (Ac-AMP) proteins discussed above (Broekaert et al,1992, Biochemistry, 31:4308-4314; International Patent Publication Number WO92/21699): all these proteins are substantially more basic than hevein or the nettle lectin which may account for the difference in activity.

We have found that possession of an overall basic profile contributes to the effectiveness of an antifungal protein. For example, in different classes of antifungal proteins isolated from Mirabilis and Raphanus it is always the more basic homologue that is the most active (Terras et al, 1992, J Biol Chem, 267: 15301-15309 ; Cammue et al, 1992, J Biol Chem, 267: 2228-2233). Although the sequence of the Capsicum (Ca-AMP1) protein is very similar to that of hevein, the ratio of basic to acidic amino acids is 4:1 for Ca-AMP1 but 4:5 (ie much lower) for hevein. The ratio of basic to acidic amino acids is 3:1 for Bm-AMP1. It may be that the basic nature of Ca-AMP1 and of Bm-AMP1 accounts for their improved potency. It is likely therefore that increasing the basic nature of certain Chitin-binding Plant Proteins (such as hevein) using site-directed mutagenesis would potentiate any antifungal activity, particularly if substitutions were made at positions where there are basic amino acids in the Capsicum (Ca-AMP1) protein (such as replacement of the aspartic acid at position 28 in hevein) or at positions where there are basic amino acids in the Briza (Bm-AMP1) protein. By adapting the structure of certain Chitin-binding Plant Proteins, it is therefore possible to create new, more potent antimicrobial proteins of the invention.

During the course of screening many different plant species it has become evident that the protein class of the invention is fairly common in plant seeds. It is possible to distinguish the proteins' antifungal activity on the basis of the unexpected morphological effect they produce: severe branching of hyphae occurs in partially inhibited germinating fungal spores. This is particularly evident when using Fusarium culmorum. We have now found that the Amaranthus protein (Ac-AMP1) causes a similar effect on fungal hyphae. The nature of the inhibition may also be characterised by the fact that it is very sensitive to the concentration of cations used in the assay.

Despite the similarities between the Capsicum protein (Ca-AMP1) and the Amaranthus proteins (Ac-AMPs), there are notable differences in their primary and tertiary structures. Figure 5 shows that the sequence of Ca-AMP1 contains at least forty-two amino acid residues. However, Ac-AMP2 is a shorter peptide: the full Ac-AMP2 sequence contains only thirty amino acid residues. Furthermore, the extra sequence of Ca-AMP1 contains two additional cysteine residues which are not found in the Ac-AMP2 protein. As cysteines are involved in internal linkages within proteins, it is probable that the tertiary structures of Ca-AMP1 and Ac-AMP2 are different.

Bm-AMP1 resembles Ca-AMP1 with respect to its total number of amino acids and its number of cysteine residues. It is probable that Bm-AMP1 and Ca-AMP1 share considerable homology at both the secondary and tertiary level. It is also probable that, like Ca-AMP1, Bm-AMP1 differs from Ac-AMP2 in its tertiary structure due in part to the two additional cysteine residues found in Bm-AMP1.

The invention further provides a recombinant DNA sequence coding for a protein of the invention, and a vector containing said sequence. The DNA may be cloned or transformed into a biological system allowing expression of the encoded protein.

In Particular, the vector may be used to transform a plant and so produce a plant transformed with recombinant DNA encoding an antimicrobial protein according to the invention.

There is also provided a process of combating fungi or bacteria, whereby they are exposed to the protein according to the invention.

Ca-AMP1 and Bm-AMP1 show a wide range of antifungal activity, and are also active against Gram-positive bacteria. Each protein is useful as a fungicide or an antibiotic, for agricultural or pharmaceutical applications. Exposure of a plant pathogen to an antimicrobial protein may be achieved by expression of the protein within a micro-organism which is applied to a plant or the soil in which a plant grows. The proteins may also be used to combat fungal or bacterial disease by application of the protein to plant parts using standard agricultural techniques (eg spraying). The proteins may also be used to combat fungal or bacterial disease by expression within plant bodies, either during the life of the plant or for post-harvest crop protection. The protein may also be used as a fungicide to treat mammalian infections.

The antimicrobial protein may be isolated and purified from appropriate seeds, synthesised artificially from its known amino acid sequence, or produced within a suitable micro-organism by expression of recombinant DNA. The proteins may also be expressed within a transgenic plant.

Amino acid sequencing of Ca-AMP1 and of Bm-AMP1 shows that they are homologous to Chitin-binding Plant Proteins. In particular Ca-AMP1 and hevein are very similar in amino acid sequence and size. Ca-AMP1 and Bm-AMP1 essentially each comprise the common cysteine/glycine domain.

Knowledge of the primary structure enables manufacture of the antimicrobial protein, or parts thereof, by chemical synthesis using a standard peptide synthesiser. It also enables production of DNA constructs encoding the antimicrobial protein. The DNA sequence may be predicted from the known amino acid sequence or the sequence may be isolated from plant-derived DNA libraries.

Oligonucleotide probes may be derived from the known amino acid sequence and used to screen a cDNA library for cDNA clones encoding some or all of the protein. These same oligonucleotide probes or cDNA clones may be used to isolate the actual antimicrobial protein gene(s) by screening genomic DNA libraries. Such genomic clones may include control sequences operating in the plant genome. Thus it is also possible to isolate promoter sequences which may be used to drive expression of the antimicrobial (or other) proteins. These promoters may be particularly responsive to environmental conditions (such as the presence of a fungal pathogen), and may be used to drive expression of any target gene.

DNA encoding the antimicrobial protein (which may be a cDNA clone, a genomic DNA clone or DNA manufactured using a standard nucleic acid synthesiser) can then be cloned into a biological system which allows expression of the protein or a part of the protein. The DNA may be placed under the control of a constitutive or inducible promoter. Examples of inducible systems include pathogen induced expression and chemical induction. Hence the protein can be produced in a suitable micro-organism or cultured cell, extracted and isolated for use. Suitable micro-organisms include Escherichia coli, Pseudomonas and yeast. Suitable cells include cultured insect cells and cultured mammalian cells. The genetic material can also be cloned into a virus or bacteriophage. The DNA can also be transformed by known methods into any plant species, so that the antimicrobial protein is expressed within the plant.

Plant cells according to the invention may be transformed with constructs of the invention according to a variety of known methods (Agrobacterium Ti plasmids, electroporation, microinjection, microprojectile gun, etc). The transformed cells may then in suitable cases be regenerated into whole plants in which the new nuclear material is stably incorporated into the genome. Both transformed monocotyledonous and dicotyledonous plants may be obtained in this way, although the latter are usually more easy to regenerate.

Examples of genetically modified plants which may be produced include field crops, cereals, fruit and vegetables such as: canola, sunflower, tobacco, sugarbeet, cotton, soya, maize, wheat, barley, rice, sorghum, tomatoes, mangoes, peaches, apples, pears, strawberries, bananas, melons, potatoes, carrot, lettuce, cabbage, onion.

The invention may be further understood by reference to the drawings, in which:
Figure 1 shows the cation exchange chromatogram for the purification of Ca-AMP1 and the associated graph of antifungal activity.
Figure 2 shows the HPLC profile of purified Ca-AMP1.
Figure 3 shows the SDS-PAGE analysis of Ca-AMP1.
Figure 4 shows the amino acid sequence of Ca-AMP1.
Figure 5 shows the alignment of the amino acid sequence of Bm-AMP1, Ca-AMP1, Ac-AMP2 and a number of chitin-binding lectins.
Figure 6 shows one possible predicted DNA sequence for the protein Ca-AMP1.
Figure 7 shows partially inhibited Fusarium culmorum spores incubated with Ca-AMP1 and the Amaranthus protein Ac-AMP1.
Figure 8 shows the SDS-PAGE analysis of different fractions after affinity chromatography of Ca-AMP1 with chitin.
Figure 9 shows the cation exchange chromatogram for the purification of Bm-AMP1 and the associated graph of antifungal activity.
Figure 10 shows the HPLC profile of purified Bm-AMP1.
Figure 11 shows the amino acid sequence of Bm-AMP1.
Figure 12 shows one possible predicted DNA sequence for Bm-AMP1.

The following examples illustrate the invention.

### EXAMPLE 1

### Antifungal and antibacterial activity assays.

Antifungal activity was measured by microspectrophotometry as previously described (Broekaert, 1990, FEMS Microbiol Lett, 69:55-60). Routinely, tests were performed with 20 µl of a (filter-sterilized) test solution and 80 µl of a suspension of fungal spores (2 x 10⁴ spores/ml) in either half strength potato dextrose broth (medium A) or half strength potato dextrose broth with CaCl₂ and RCl added to final concentrations of 1 mM and 50 mM respectively (medium B).

Unless otherwise stated the test organism was Fusarium culmorum (strain IMI 180420) and incubation was done at 25°C for 48 hours. Percent growth inhibition is defined as 100 times the ratio of the corrected absorbance of the control microculture minus the corrected absorbance of the test microculture over the corrected absorbance at 595 nm of the control microculture. The corrected absorbance values equal the absorbance at 595 nm of the culture measured after 48 hours minus the absorbance at 595 nm measured after 30 min.

Antibacterial activity was measured microspectrophotometrically as follows. A bacterial suspension was prepared by inoculating soft nutrient agarose (tryptone, 10 g/l; Seaplaque agarose (FMC), 5 g/l). Aliquots (80 µl) of the bacterial suspension (10⁵ colony forming units per ml) were added to filter-sterilized samples (20 µl) in flat-bottom 96-well microplates. The absorbance at 595 nm of the culture was measured with the aid of a microplate reader after 30 minutes and 24 hours of incubation at 28°C. Percent growth inhibition was calculated as described above for the antifungal activity assay.

### EXAMPLE 2

### Extraction of basic proteins from Capsicum annuum or Briza maxima seeds

One kilogramme of Capsicum annuum or Briza maxima seeds (from Chiltern seeds, Cumbria, UK) were ground in a coffee mill and the resulting meal was extracted for 2 hours at 4°C with 2 litres of an ice-cold extraction buffer containing 10 mM NaH₂PO₄, 15 mM Na₂HPO₄, 100 mM KCl, 2 mM EDTA and 1 mM benzamidine. The resulting homogenate was squeezed through cheesecloth and clarified by centrifugation (30 min at 7,000 x g). Solid ammonium sulphate was added to the supernatant to obtain 75% relative saturation and the precipitate allowed to form by standing overnight at 4°C. Following centrifugation at 7,000 x g for 30 minutes, the precipitate was redissolved in a minimal volume of distilled water and dialyzed extensively against distilled water using benzoylated cellulose tubing (Sigma, St Louis, MO). After dialysis the solution was adjusted to 50 mM NH₄Ac (pH 9) by addition of the ten-fold concentrated buffer and passed over a Q-Sepharose Fast Flow (Pharmacia, Uppsala, Sweden) column (12 x 5 cm) equilibrated in 50 mM NH₄Ac (pH 9). The protein fraction which passed through the column was adjusted to pH6 with acetic acid.

This material represents the basic (pI>9) protein fraction of the seeds. The fractions were further purified as described in Example 3.

### EXAMPLE 3

### Purification of antimicrobial protein from Capsicum annuum or Briza maxima seeds

The starting material for the isolation of the C annuum or B maxima antimicrobial protein was the basic protein fraction extracted from the mature seeds as in Example 2. Proteins were further purified by cation exchange chromatography of this extract.

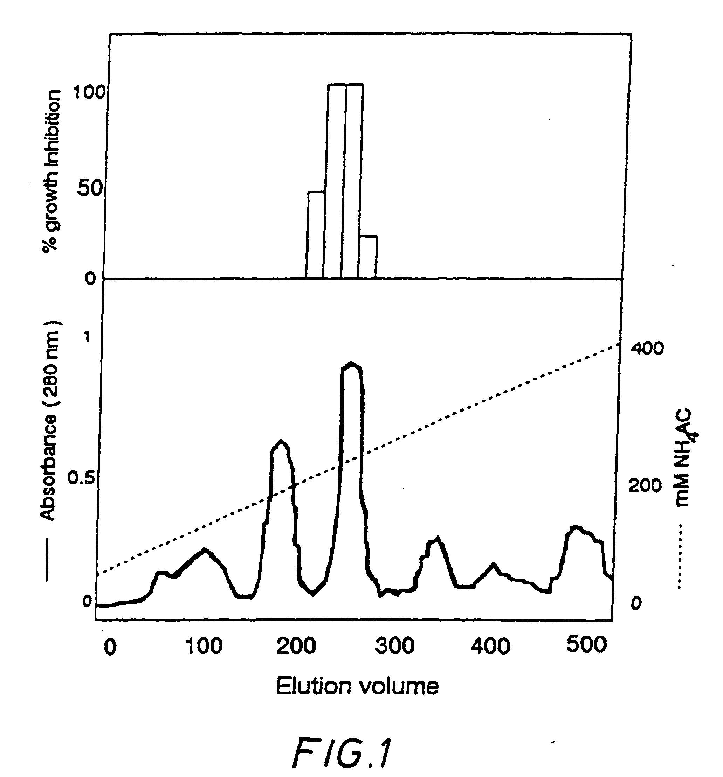

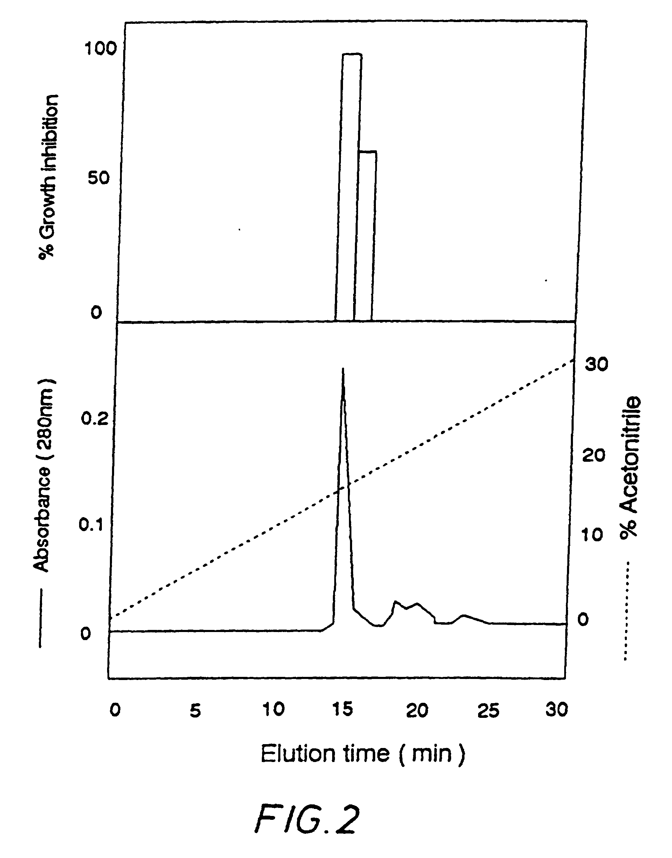

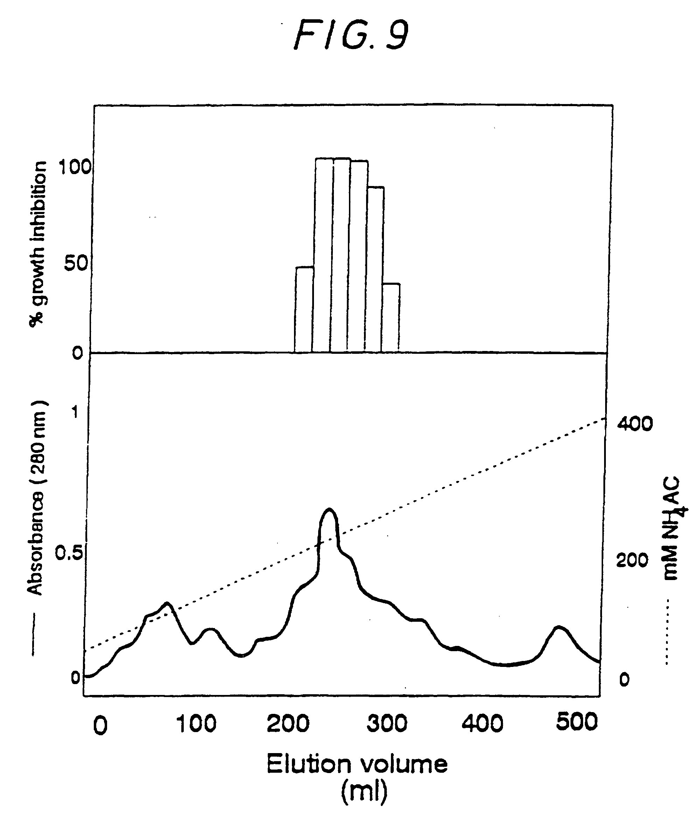

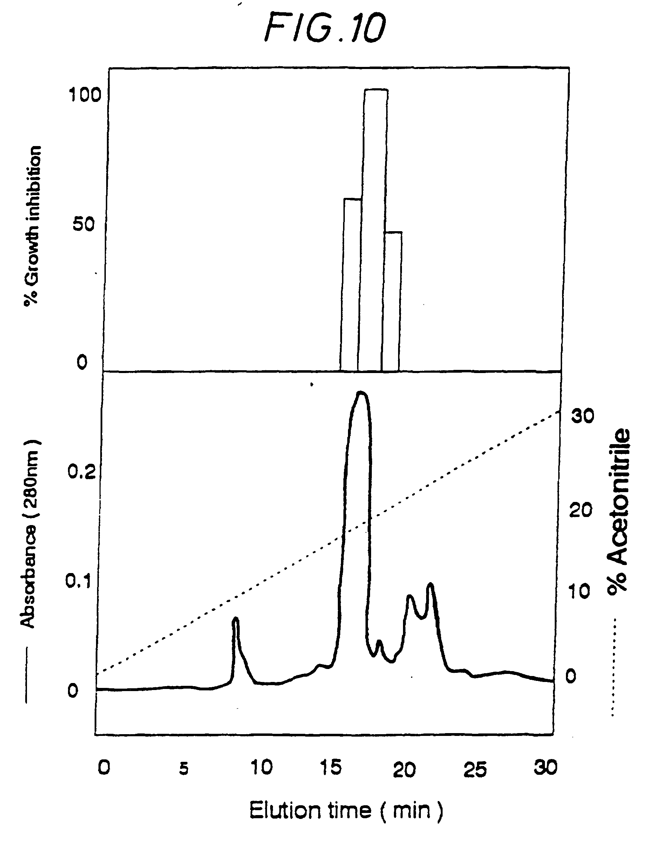

Approximately 500 ml of the basic protein fraction was applied to a S-Sepharose High Performance (Pharmacia) column (10 x 1.6 cm) equilibrated in 50 mM NH₄Ac, pH 6.0. The column was eluted at 3.0 ml\min with a linear gradient of 50-750 mM NH₄Ac, pH 6.0 over 325 minutes. The eluate was monitored for protein by online measurement of the absorbance at 280 nm (results for Capsicum and for Briza shown in the lower panels of Figures 1 and 9 respectively) and collected in 10 ml fractions. Samples from each fraction were assayed for antifungal activity as described in Example 1 (results for Capsicum and for Briza shown in the upper panels of Figures 1 and 9 respectively). Following chromatography, the Capsicum extract yielded a broad peak of activity eluting at around 220 mM NH₄Ac. The Briza extract yielded a broad peak of activity eluting at around 250 mM NH₄Ac. The fractions showing antifungal activity were pooled and further purified by reverse-phase HPLC. About 3 mg amounts of the peak were loaded on a PEP-S (porous silica C₂/C₁₈, Pharmacia) column (25 x 0.4 cm) equilibrated with 0.1% TFA (trifluoracetic acid). The column was developed at 1 ml/min with a linear gradient of 0.1% TFA to 100% acetonitrile/0.1% TFA over 100 minutes. The eluate was monitored for protein by online measurement of the absorption at 280 nm (results for Capsicum and for Briza shown in the lower panels of Figures 2 and 10 respectively). One ml fractions were collected, vacuum dried, and redissolved in lml distilled water of which 10µl was used in an anti-fungal assay (results for Capsicum and for Briza shown in the upper panels of Figures 2 and 10 respectively). The single well-resolved peaks of activity were called Ca-AMP1 and Bm-AMP1 respectively.

### EXAMPLE 4

### Molecular structure of the purified antimicrobial protein Ca-AMP1.

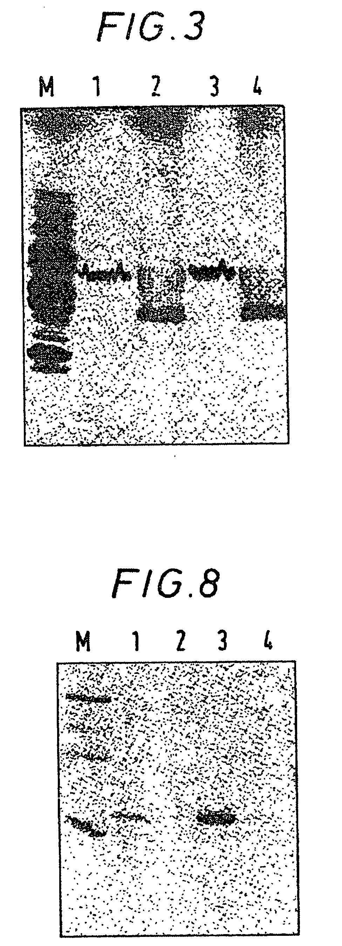

The molecular structure of the purified antimicrobial protein was further analysed. Sodium dodecyl sulphate polyacrylamide gel electrophoresis (SDS-PAGE) was performed on precast commercial gels (PhastGel High Density from Pharmacia) using a PhastSystem (Pharmacia) electrophoresis apparatus. The sample buffer contained 200 mM Tris-HCl (pH 8.3), 1% (w/v) SDS, mM EDTA, 0.005% bromophenol blue and, unless otherwise stated, 1% (w/v) dithioerythritol (DTE). Proteins were fixed after electrophoresis in 12.5% glutaraldehyde and silver-stained according to Heukeshoven and Dernick (1985, Electrophoresis, 6, 103-112). Molecular weight markers (Pharmacia) were run for comparison (lane M, Figure 3): 17 kDa, 14.5 kDa, 8 kDa, 6 kDa, 2.5 kDa.

Ca-AMP1 was analysed by SDS-PAGE. After reduction with β-mercaptoethanol, Ca-AMP1 runs as a single band with an apparent molecular mass of 4 to 5 kDa (Figure 3, lane 2 and 4). Unreduced Ca-AMP1 migrates as a single band of 14 kDa (Figure 3, lane 1 and 3). These results show that the native Ca-AMP1 is in oligomeric protein, probably a dimer.

### EXAMPLE 5

### Amino acid sequencing of Ca-AMP1 and Bm-AMP1.

Cysteine residues were modified by S-pyridylethylation using the method of Fullmer (1984, Anal Biochem, 142, 336-341). Reagents were removed by HPLC on a Pep-S (porous silica C₂/C₁₈) (Pharmacia) column (25 x 0.4 cm). The S-pyridylethylated proteins were recovered by eluting the column with a linear gradient from 0.1 % trifluoroacetic acid (TFA) to acetonitrile containing 0.1 % TFA. The resulting protein fractions were subjected to amino acid sequence analysis in a 477A Protein Sequencer (Applied Biosystems) with on-line detection of phenylthiohydantoin amino acid derivatives in a 120A Analyser (Applied Biosystems).

Initial attempts to sequence Ca-AMP1 showed that the protein was N-terminally blocked. Subsequently, the S-pyridylethylated protein was unblocked with pyroglutamate amino peptidase according to the supplier's instructions (Boehringer Mannheim, FRG). The reaction was only partially successful and yielded sequence for the first 16 amino acids.

In order to obtain sequence for the C-terminus, Ca-AMP1 was digested with trypsin and three of the resulting fragments were sequenced. One was found to be blocked and represents the N-terminus. Sequencing of the other two peptides showed that they could be aligned with the sequence for the N-terminus (Figure 4) and that the complete sequence was homologous to the cysteine/glycine-rich domain found in chitin-binding plant lectins (Figure 5). It is possible that the sequence for Ca-AMP1 is incomplete and that there are more amino acids at the C-terminus. The finding that the peptide was N-terminally blocked and that this could be removed with aminopeptidase suggests that the N-terminal amino acid may be a glutamine.

The amino acid sequence of Bm-AMP1 is shown in Figure 11. At two positions in the sequence there is a choice of two amino acids. At position 9 the amino acid is either arginine (R) or histidine (H), and at position 23 the amino acid is either serine (S) or asparagine (N). The purified protein fraction Bm-AMP1 may be a mixture of peptides having sequences varying at these two positions with any combination of the stated amino acids.

Figure 5 shows the alignment of N-terminal amino acid sequences from tobacco chitinase (Shinshi et al, 1987, Proc Nat Acad Sci USA, 84:89-93), bean chitinase (Broglie et al, 1986, Proc Nat Acad Sci USA, 83:6820-6824), hevein (Broekaert et al, 1990, Pro Nat Acad Sci USA, 87:7633-7637), wheat lectin (Raikhel and Nilkins, 1987, Proc Nat Acad Sci USA, 84:6745-6749), nettle lectin (Chapot et al, 1986, FEBS Lett, 195:231-234), Ac-AMP2 (Broekaert et al, 1992, Biochemistry, 31:4308-4314; International Patent Publication Number WO92/21699) and the sequences for Ca-AMP1 and Bm-AMP1. Sequence identities and conserved changes are boxed. Conserved changes are considered as substitutions within the amino acid homology groups FWY, MILV, RKH, ED, NQ, ST and PAG. Gaps introduced for maximum alignment are represented by dashes.

The amino acid sequence for Ca-AMP1 and for Bm-AMP1 shows striking similarity to the cysteine/glycine rich domain in Chitin-binding Plant Proteins. In particular, Ca-AMP1 is 65% identical to hevein. Bm-AMP1 is 35% to hevein and 45% identical to Ca-AMP1. Like the Amaranthus proteins, Ca-AMP1 and Bm-AMP1 are substantially more basic than hevein.

Both Ca-AMP1 and hevein have four basic amino acids, but Ca-AMP1 has only one acidic amino acid compared to five in hevein. If the overall basic nature of these proteins is important for their activity then substitutions of the aspartic acid at position 28 in hevein for the arginine found at this position in Ca-AMP1 would be expected to increase the specific activity of hevein. Indeed, it seems quite remarkable that Ca-AMP1 and hevein are so similar in size and amino acid sequence, but differ so dramatically in their levels and spectrum of activity.

Bm-AMP1 contains six basic amino acids and only two acidic amino acids, whereas hevein has four basic amino acids but five acidic amino acids. The overall basic profile of Bm-AMP1 may therefore be related to the increased antifungal activity of this protein compared to hevein and other Chitin-binding Plant Proteins.

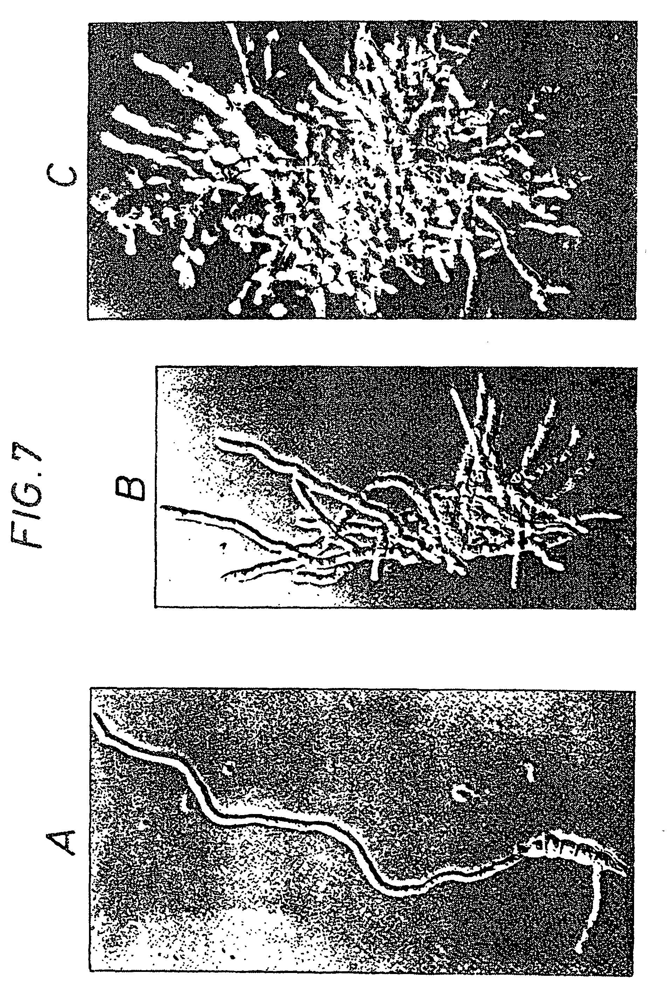

Figure 6 and Figure 12 show one of the possible DNA sequences of the gene coding for Ca-AMP1 and Bm-AMP1 respectively. This gene sequence has been predicted from the known amino acid sequence using codons which commonly occur in dicotyledonous plants. The actual gene sequence within Capsicum or Briza many differ due to the degeneracy of the genetic code.

### EXAMPLE 6

### Stability of the protein's antifungal activity.

Tests for antifungal activity were performed with 20 µl samples diluted five-fold with growth medium containing Fusarium culmorum spores, according to the assay method given in Example 1. Untreated control samples consisted of the test proteins at 500 µg/ml in 10 mM sodium phosphate buffer (pH 7). Heat stability tests were performed by heating aliquots of the test proteins for 10 minutes at different temperatures up to 100°C. Reduction of disulphide bridges was done by addition of dithiothreitol at 30 mM and Tris-HCl (pH 8.6) at 300 mM. The reagents were removed by reversed-phase chromatography. For digestions, different proteases were added at 200 µg/ml and incubated at 37°C for 3 hours. The control treatment containing only the reagents proved negative for antifungal activity after the reversed-phase chromatography step.

The antifungal activity of the purified Ca-AMP1 protein and the Bm-AMP1 protein was resistant to heat treatment at up to 80°C for 10 minutes. Reduction of the disulphide bonds by dithiothreitol, however, completely abolished the antifungal activity. These disulphide linkages are essential for biological activity.

Treatment of Ca-AMP1 with proteinase K or pronase E reduced the antifungal activity by at least 10-fold, whereas trypsin only reduced the activity by 2-fold and chymotrypsin had no affect on activity.

### EXAMPLE 7

### Antifungal potency of the proteins.

### (A) Ca-AMP1.

The antifungal potency of the purified protein was assessed on different plant pathogenic fungi, using the assay described in Example 1. Growth of fungi, collection and harvest of fungal spores, and preparation of mycelial fragments were done as previously described (Broekaert et al, 1990, FEMS Microbiol Lett, 69:55-60). The following fungal strains were used: Alternaria brassicola MUCL 20297, Ascochyta pisi MUCL 30164, Botrytis cinerea MUCL 30158, Cercospora beticola strain K897, Colletotrichum lindemuthianum MUCL 9577, Fusarium culmorum IMI 180420, Fusarium oxysporum f.sp. pisi IMI 236441, Fusarium oxysporum f.sp. lycopersici MUCL 909, Nectria haematococca Collection Van Etten 160-2-2, Penicillium digitatum (K0879), Phoma betae MUCL 9916, Pyrenophora tritici-repentis MUCL 30217, Pyricularia oryzae MUCL 30166, Rhizoctonia solani CBS 207-84, Septoria tritici (K1097D), Trichoderma viride (K1127), Verticillium albo-atrum (K0937), Verticillium dahliae MUCL 19210.

For R solani, mycelial fragments were used as inoculum, whereas all other fungi were inoculated as spores.

Serial dilutions of the antifungal proteins were applied to the fungi, either using growth medium A or medium B. The percent growth inhibition was measured by microspectrophotometry. The concentration required for 50% growth inhibition after 48 h of incubation (IC₅₀ value) was calculated from the dose-reponse curves.

Results are summarised in Table 1.

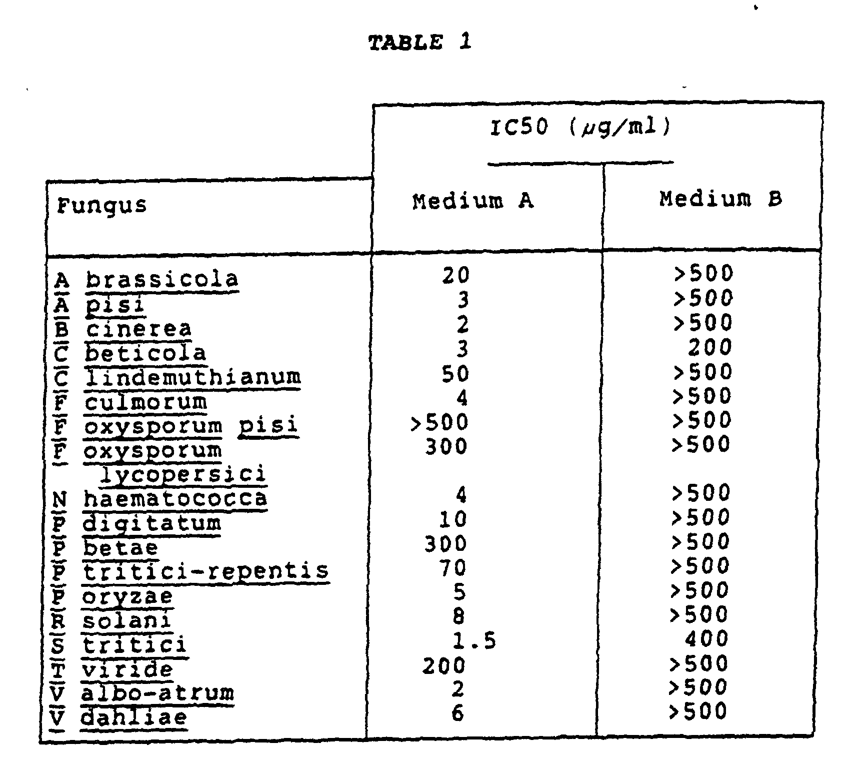

Assayed on a range of fungi in medium A the IC₅₀ values varied from 1 µg/ml to over 500 µg/ml. However, for 12 of the 18 pathogenic fungi the IC₅₀ value was below 50 µg/ml and for 10 of the fungi the IC₅₀ value was below 10 µg/ml. The results show that Ca-AMP1 is a potent and broad spectrum inhibitor of fungal growth.

The activity of Ca-AMP1 is, however, very sensitive to the ionic conditions used in the assay and it's activity is essentially abolished in high salt (medium B).

The level of antifungal activity obtained with Ca-AMP1 is comparable to that of two peptides (Ac-AMPs) previously isolated from Amaranthus seeds (Broekaert et al, 1992, Biochemistry, 31:4308-4314). Relative to Chitin-binding Plant Proteins, such as hevein or nettle lectin, Ca-AMP1 has much higher specific activity. Previously we have shown that nettle lectin inhibits only 3 of 7 fungi tested at concentrations below 100 µg/ml and none below 20 µg/ml (Broekaert et al, 1992, Biochemistry 31:4308-4314). Similarly, hevein has been reported to be much less active than even nettle lectin (Van Parijs et al, 1991, Planta 183:258-264). Despite the similarity in amino acid sequence, therefore, Ca-AMP1 can, like the Amaranthus proteins, be classed separately from the Chitin-binding Plant Proteins.

Ca-AMP1 and the Amaranthus proteins give rise to the same morphological changes in partially inhibited fungal spores. This is readily visualised when Fusarium culmorum spores are used in the assay and at concentrations of the proteins which are 2-4 fold below the IC₅₀ value. Viewed under a light microscope, the proteins cause severe branching of the emerging hyphal tips (Figure 7). Figure 7A shows control spores germinated for 8 hours at 24°C; Figure 7B and 7C show spores partially inhibited by Ca-AMP1 and Ac-AMP1 respectively. Hevein has been reported to cause the development of thick hyphae and buds (Van Parijs et al, 1991, Planta, 183:258-264).

### (B) Bm-AMP1.

The antifungal potency of the purified protein was assessed on different plant pathogenic fungi, using the assay described in Example 1. Growth of fungi, collection and harvest of fungal spores, and preparation of mycelial fragments were done as previously described (Broekaert et al, 1990, FEMS Microbiol Lett, 69:55-60). The following fungal strains were used: Alternaria longipes strain CBS 620.83, Botrytis cinerea MUCL 30158, Cladosporium sphaerospermum strain KO791, Fusarium culmorum IMI 180420, Penicillium digitatum strain K0879, Septoria tritici (K1097D), Trichoderma viride (K1127), Verticillium dahliae MUCL 19210.

All fungi were inoculated as spores.

Serial dilutions of the antifungal proteins were applied to the fungi, either using growth medium A or medium B. The percent growth inhibition was measured by microspectrophotometry. The concentration required for 50% growth inhibition after 48 h of incubation (IC₅₀ value) was calculated from the dose-reponse curves.

The results for Bm-AMP1 are summarised in Table 2.

**TABLE 2**

| Fungus | IC50 (µg/ml) | |
|---|---|---|
| | Medium A | Medium B |
| A longipes | 2 | >500 |
| B cinerea | 9 | >500 |
| C sphaerospermum | 3 | >500 |
| F culmorum | 9 | >500 |
| P digitatum | 6 | >500 |
| S tritici | 1 | 400 |
| T viride | 150 | >500 |
| V dahliae | 10 | >500 |

Assayed on a range of fungi in medium A the IC₅₀ values varied from 1 µg/ml to 150 µg/ml. However, for six of the eight pathogenic fungi the IC₅₀ value was below 10 µg/ml. The results show that Bm-AMP1 is a potent and broad spectrum inhibitor of fungal growth.

The activity of Bm-AMP1 is, however, very sensitive to the ionic conditions used in the assay and it's activity is essentially abolished in high salt (medium B).

The level and spectrum of antifungal activity obtained with Bm-AMP1 is comparable to that of Ca-AMP1 and to that of the two peptides (Ac-AMPs) previously isolated from Amaranthus seeds. Relative to Chitin-binding Plant Proteins, such as hevein or nettle lectin, Bm-AMP1 has much higher specific activity. Previously we have shown that nettle lectin inhibits only 3 of 7 fungi tested at concentrations below 100 µg/ml and none below 20 µg/ml (Broekaert et al, 1992, Biochemistry 31:4308-4314). Similarly, hevein has been reported to be much less active than even nettle lectin (van Parijs et al, 1991, Planta 183:258-264). Despite the similarity in amino acid sequence, therefore, Bm-AMP1 can, like Ca-AMP1 and the Amaranthus proteins, be classed separately from the Chitin-binding Plant Proteins.

### EXAMPLE 8

### Chitin-binding Activity of Ca-AMP1.

The similarity in sequence between Ca-AMP1 and chitin-binding plant lectins suggested that Ca-AMP1 might also bind to chitin.

Micro-chitin-columns were loaded with Ca-AMP1 and the columns washed with 50mM NH₄Ac (pH 7.0). 50µg Ca-AMP1 was loaded onto the column (0.5 x 1cm) and the eluate recycled over the column three times. The final eluate was collected. The column was washed five times with 1ml 50mM NH₄Ac (pH 7.0) and this fraction collected. Finally, the column was washed with 1ml 100mM acetic acid (pH 2.8) and this acid-wash fraction collected. The collected fractions were desalted and concentrated by reverse-phase chromatography and finally dissolved in 50µl sample buffer for SDS-PAGE analysis.

Figure 8 shows the results of this analysis. Lane M shows molecular weight markers of 29 kDa, 20.1 kDa, 13.2 kDa and 5 kDa in size. Lane 1 is reduced Ca-AMP1 run as a control. Lane 2 is the fraction eluted with repeated 50mM NH₄Ac (pH 7.0) washes. Lane 3 is the acid-wash fraction and lane 4 the initial flow-through. It can be seen that the majority of the protein binds to the column and is eluted in the low pH desorption buffer, suggesting that Ca-AMP1 exhibits affinity to chitin.

### EXAMPLE 9

### Anti-bacterial and anti-yeast activity of Ca-AMP1 and of Bm-AMP1.

The purified proteins were assessed for effect on the growth of the following bacteria: Bacillus megaterium ATCC 13632, Escherichia coli strain HB101 and Pseudomonas aeurogenasa NCIB 8295; and for its effect on the growth of Saccharomyces cerevisiae JRY188. Bioassays were carried out as described in Example 1. The results are summarised in Table 3. Ca-AMP1 and Bm-AMP1 each strongly inhibited the growth of B megaterium and S cerevisiae but had little or no effect on the two Gram negative bacteria tested.

**TABLE 3**

| **Activity of Ca-AMP1 and Bm-AMP1 on bacteria and yeast** | | |
|---|---|---|
| | IC50 (µg/ml) | |
| | Ca-AMP1 | Bm-AMP1 |
| B megaterium | 20 | 10 |
| P aeurogenasa | 500 | 500 |
| E coli | >800 | >800 |
| S cerevisiae | 30 | 15 |

### EXAMPLE 10

### Molecular cloning of Ca-AMP1 and Bm-AMP1 cDNAs

From outdoor grown plants, seeds at 6 different developmental stages are collected, frozen in liquid nitrogen and stored at -80°C. After pulverisation, total RNA is extracted from 15 g of a mixture of the 6 different developmental stages, using the method of De Vries et al (1988, Plant Molecular Biology Manual, B6, 1-13) with the exception that 6 ml of a 1:2 phenol:RNA extraction buffer mixture and 2 ml of chloroform are used per g of tissue. Poly (A)⁺ mRNA is purified by affinity chromatography on oligo(dT)-cellulose as described by Siflow et al (1979, Biochemistry 18, 2725-2731). Double stranded cDNAs are prepared from 1.5 µg of poly(A)⁺ RNA according to Gubler and Hoffman (1983, Gene 25, 263-269) and ligated to EcoRI/NotI adaptors using the cDNA Synthesis Kit of Pharmacia.

The cDNAs are cloned into the lambda ZAPII phage vector (Stratagene) according to the manufacturers instructions. A DNA probe for screening the cDNA library is produced by polymerase chain reaction (PCR) as follows. Two degenerate oligonucleotides are synthesised, corresponding to a run of amino acids of Ca-AMP1 or Bm-AMP1: one has a sense orientation and the other has an antisense orientation. Both primers have the AAAGAATTC (i.e. AAA followed by the EcoRI recognition sequence) sequence at their 5' ends. PCR is performed with the Taq polymerase under standard conditions (Sambrook et al, 1989, Molecular Cloning, Cold Spring Harbor Laboratory Press) using the oligonucleotides as amplimers and 25 ng of cDNA as target DNA. The temperature programme includes an initial step at 94°C for 5 min, 30 cycles (94°C for 1 min; 45°C for 2 min, 72°C for 3 min) and a final step at 72°C for 10 min. The PCR amplification product is purified on a 3% agarose (NuSieve, FMC) gel. This PCR product is partially reamplified using degenerate oligonucleotides. This PCR amplification product is again purified on a 3% agarose (NuSieve, FMC) gel and reamplified by PCR under the same conditions except that the reaction mixture contained 130 µM dTTP and 70 µM digoxigenin-11-dUTP instead of 200 µM dTTP. The digoxigenin-labeled PCR product is purified on a 3% NuSieve agarose gel. About 10,000 plaque forming units of the lambda ZAPII cDNA library are screened with the digoxigenin-labeled PCR product by in situ plaque hybridisation using nylon membranes (Hybond-N, Amersham). Membranes are air-dried and DNA is crosslinked to the membranes under UV light (0.15 J/cm²). Hybridisation is performed for 16 h at 64°C in 5 x SSC, 1 % blocking reagent (Boehringer Mannheim), 0.1 % N-lauroylsarcosine, 0.02 % sodium dodecylsulphate containing 10 ng/ml of heat denatured digoxigenin-labeled probe. Non-specifically bound probe is removed by rinsing two times 5 min in 2 x SSC / 0.1 % SDS at 25°C and two times 15 min in 0.1 x SSC / 0.1 % SDS at 60°C. Detection of the probe is done using anti-digoxigenin antibodies linked to alkaline phosphatase (Boehringer Mannheim) and its substrate 5-bromo-4-chloro-3-indolyl phosphate (Boehringer Mannheim) according to the manufacturers instructions. Positive plaques are purified by two additional screening rounds with the same probe under the same conditions. Inserts from purified plaques are excised in vivo into the pBluescript phagemid form with the aid of the helper phage R408. The inserts from different positive clones are excised by EcoRI digestion and their sizes compared by agarose gel electrophoresis. Some of the clones are subjected to nucleotide sequence analysis. The clones with the largest insert may have an open reading frame corresponding to Ca-AMP1 or Bm-AMP1, as could be determined by comparison to the experimental N-terminal amino acid sequences. The full-length cDNA clones may differ from each other in the length of their 5' and 3' end untranslated regions and polyadenylation signals.

In order to obtain a full-length cDNA, another approach may be followed. PCR is performed under standard conditions using an antisense oligonucleotide in combination with the M13 universal primer at one hand and the M13 reverse primer at the other hand. The last nucleotides of the oligonucleotide form the inverted complementary sequence of part of the 3' untranslated region immediately flanking the poly-A tail of the less-than-full-length cDNA clone. This sequence is extended to the 5' end with the GAATTC EcoRI recognition site preceded by the nucleotides 'ATA'. As a template, either 2 µg of total cDNA or 10⁵ recombinant phages are used. In both cases, 3 separate reactions are set up. Prior to amplification, phages are lysed by an initial step in the PCR temperature programme of 5 min at 99°C to liberate the phage DNA. The size of the amplification products is determined by electrophoresis on a 3% agarose (NuSieve, FMC) gel. Products are obtained with sizes corresponding to inserts of different length, including a full-length cDNA clones if one is present in the cDNA library.

### EXAMPLE 11

### Construction of an expression vector

An expression vector is constructed, containing the full coding region of the Ca-AMP1 or Bm-AMP1 DNA flanked at its 5' end by the strong constitutive promoter of the 35S RNA of the cauliflower mosaic virus (Odell et al, 1985, Nature 313, 810-812) with a duplicated enhancer element to allow for high transcriptional activity (Kay et al, 1987, Science 236, 1299-1302). The coding region of the Ca-AMP1/Bm-AMP1 DNA is flanked at its 3' end side by the polyadenylation sequence of 35S RNA of the cauliflower mosaic virus (CaMV35S). The plasmid backbone of this vector is the phagemid pUC120 (Vieira and Messing 1987, Methods Enzymol. 153, 3-11). The expression vector is constructed as follows. A cDNA clone consisting of the Ca-AMP1/Bm-AMP1 DNA is cloned into the BamHI / SalI sites of pEMBL18+, Boehringer). The BamHI / SalI fragment is subcloned into the expression vector pFAJ3002 which was pre-digested with BamHI and SalI. pFAJ3002 is a derivative of the expression vector pFF19 (Timmermans et al, 1990, J. Biotechnol. 14, 333-344) of which the unique EcoRI site is replaced by a HindIII site.

### EXAMPLE 12

### Construction of a plant transformation vector

The expression vector from Example 11 is digested with HindIII and the fragment containing the Ca-AMP1/Bm-AMP1 DNA expression cassette is subcloned into the unique HindIII site of pBin19Ri. pBin19Ri is a modified version of the plant transformation vector pBin19 (Bevan 1984, Nucleic Acids Research 12, 8711-8721) wherein the unique EcoRI and HindIII sites are switched and the defective nptII expression cassette (Yenofsky et al. 1990, Proc. Natl. Acad. Sci. USA 87: 3435-3439) is introduced.

### EXAMPLE 13

### Plant Transformation

The disarmed Agrobacterium tumefaciens strain LBA4404 (pAL4404)(Hoekema et al, 1983, Nature 303, 179-180) is transformed with the vector made in Example 12 using the method of de Framond et al (BioTechnology 1, 262-269).

Tobacco transformation is carried out using leaf discs of Nicotiana tabacum Samsun based on the method of Horsch et al (1985, Science 227, 1229-1231) and co-culturing with Agrobacterium strains containing pFRG8. Co-cultivation is carried out under selection pressure of 100 µg/ml kanamycin. Transgenic plants (transformed with pFRG8) are regenerated on media containing 100 µg/ml kanamycin. These transgenic plants may be analysed for expression of the newly introduced genes using standard western blotting techniques. Plants capable of constitutive expression of the introduced genes may be selected and self pollinated to give seed. Fl seedlings of the transgenic plants may be further analysed.

## Claims

1. An antimicrobial protein comprising the amino acid sequence shown in Figure 4 or in Figure 11.

2. A protein as claimed in claim 1 which is an oligomer and which comprises at least one polypeptide having the amino acid sequence shown in Figure 4 or in Figure 11.

3. A protein as claimed in claim 1 wherein said protein consists of the amino acid sequence shown in Figure 4 which is the pure protein Ca-AMP1.

4. A protein as claimed in claim 1 wherein said protein consists of the amino acid sequence shown in Figure 11 which is the pure protein Bm-AMP1.

5. A protein as claimed in claim 1 which is capable of being isolated from a plant seed.

6. A protein as claimed in claim 5 which is capable of being isolated from seed selected from the group consisting of Capsicum seed, Briza seed, Catapodium seed, Baptisia seed, Microsensis seed, Delphinium seed.

7. A protein as claimed in claim 1 which is synthetic.

8. A protein as claimed in claim 1 which is produced by expression of recombinant DNA.

9. A composition suitable for application to plant parts using standard agricultural techniques and containing a protein as claimed in any one of claims 1 to 8.

10. A recombinant DNA sequence encoding a protein as claimed in any of claims 1 to 6.

11. A DNA sequence as claimed in claim 10 which is cDNA.

12. A DNA sequence as claimed in claim 10 which is genomic DNA.

13. A DNA sequence as claimed in claim 10 which is isolated from a plant genome.

14. A vector containing a DNA sequence as claimed in any one of claims 10 to 13.

15. A process of combating fungal or bacterial infection in a plant which process comprises applying to said plant a protein according to any of claims 1 to 8.

16. A process as claimed in claim 15 which comprises exposure to a composition as claimed in claim 9.

17. An extraction process for producing a protein as claimed in any of claims 1 to 6 from organic material containing them which comprises submitting the organic material to maceration and solvent extraction.

18. An extraction process as claimed in claim 17 where the protein is subsequently purified by centrifugation, chromatography and dialysis.

19. An extraction process as claimed in claim 17 where the organic matter comprises a seed selected from the group consisting of Capsicum seed, Briza seed, Catapodium seed, Baptisia seed, Microseasis seed, Delphinium seed.

20. A process for producing a protein as claimed in any of claims 1 to 6 which comprises chemical synthesis of the protein.

## Patentansprüche

1. Antimikrobielles Protein, das die in Fig. 4 oder Fig. 11 gezeigte Aminosäuresequenz umfasst.

2. Protein gemäss Anspruch 1, das ein Oligomer ist und wenigstens ein Polypeptid mit der in Fig. 4 oder Fig. 11 gezeigten Aminosäuresequenz umfasst.

3. Protein gemäss Anspruch 1, worin das Protein aus der in Fig. 4 gezeigten Aminosäuresequenz besteht, die das reine Protein Ca-AMP1 ist.

4. Protein gemäss Anspruch 1, worin das Protein aus der in Fig. 11 gezeigten Aminosäuresequenz besteht, die das reine Protein Bm-AMP1 ist.

5. Protein gemäss Anspruch 1, das aus einem Pflanzensamen isoliert werden kann.

6. Protein gemäss Anspruch 5, das aus Samen isoliert werden kann, der aus der Gruppe ausgewählt ist, die aus Capsicum-Samen, Briza-Samen, Catapodium-Samen, Baptisia-Samen, Microsensis-Samen und Delphinium-Samen besteht.

7. Protein gemäss Anspruch 1, das synthetisch ist.

8. Protein gemäss Anspruch 1, das durch Expression von rekombinanter DNA erzeugt wird.

9. Zusammensetzung, die zur Ausbringung auf Pflanzenteile unter Verwendung von landwirtschaftlichen Standardtechniken geeignet ist und ein Protein gemäss einem der Ansprüche 1 bis 8 enthält.

10. Rekombinante DNA-Sequenz, die ein Protein gemäss einem der Ansprüche 1 bis 6 codiert.

11. DNA-Sequenz gemäss Anspruch 10, die cDNA ist.

12. DNA-Sequenz gemäss Anspruch 10, die genomische DNA ist.

13. DNA-Sequenz gemäss Anspruch 10, die aus einem pflanzlichen Genom isoliert ist.

14. Vektor, der eine DNA-Sequenz gemäss einem der Ansprüche 10 bis 13 enthält.

15. Verfahren zur Bekämpfung von pilzlicher oder bakterieller Infektion in einer Pflanze, wobei das Verfahren das Ausbringen eines Proteins gemäss einem der Ansprüche 1 bis 8 auf die Pflanze umfasst.

16. Verfahren gemäss Anspruch 15, das den Kontakt mit einer Zusammensetzung gemäss Anspruch 9 umfasst.

17. Extraktionsverfahren zur Herstellung eines Proteins gemäss einem der Ansprüche 1 bis 6 aus organischem Material, das es enthält, wobei das Verfahren das Unterwerfen des organischen Materials der Mazeration und Lösungsmittelextraktion umfasst.

18. Extraktionsverfahren gemäss Anspruch 17, worin das Protein anschliessend durch Zentrifugieren, Chromatografie und Dialyse gereinigt wird.

19. Extraktionsverfahren gemäss Anspruch 17, worin das organische Material einen Samen umfasst, der aus der Gruppe ausgewählt ist, die aus Capsicum-Samen, Briza-Samen, Catapodium-Samen, Baptisia-Samen, Microsensis-Samen und Delphinium-Samen besteht.

20. Verfahren zur Herstellung eines Proteins gemäss einem der Ansprüche 1 bis 6, welches die chemische Synthese des Proteins umfasst.

## Revendications

1. Protéine antimicrobienne comprenant la séquence d'aminoacides présentée dans la figure 4 ou dans la figure 11.

2. Protéine selon la revendication 1, qui est un oligomère et qui comprend au moins un polypeptide ayant la séquence d'aminoacides présentée dans la figure 4 ou dans la figure 11.

3. Protéine selon la revendication 1, ladite protéine étant constituée par la séquence d'aminoacides présentée dans la figure 4, qui est la protéine Ca-AMP1 pure.

4. Protéine selon la revendication 1, ladite protéine étant constituée par la séquence d'aminoacides présentée dans la figure 11, qui est la protéine Bm-AMP1 pure.

5. Protéine selon la revendication 1, qu'il est possible d'isoler de la graine d'une plante.

6. Protéine selon la revendication 5, qu'il est possible d'isoler de graines choisies dans le groupe constitué par la graine de *Capsicum,* la graine de *Briza,* la graine de *Catapodium,* la graine de *Baptisia,* la graine de *Microsensis,* la graine de *Delphinium.*

7. Protéine selon la revendication 1, qui est synthétique.

8. Protéine selon la revendication 1, qui est produite par l'expression d'un ADN recombiné.

9. Composition appropriée à une application à des parties végétales au moyen de techniques standards de l'agriculture et contenant une protéine selon l'une quelconque des revendications 1 à 8.

10. Séquence d'ADN recombiné codant pour une protéine selon l'une quelconque des revendications 1 à 6.

11. Séquence d'ADN selon la revendication 10, qui est un ADNc.

12. Séquence d'ADN selon la revendication 10, qui est un ADN génomique.

13. Séquence d'ADN selon la revendication 10, qui est isolé d'un génome de plante.

14. Vecteur contenant une séquence d'ADN selon l'une quelconque des revendications 10 à 13.

15. Procédé de lutte contre les infections fongiques et bactériennes dans une plante, ce procédé comprenant l'application, à ladite plante, d'une protéine selon l'une quelconque des revendications 1 à 8.

16. Procédé selon la revendication 15, qui comprend l'exposition à une composition selon la revendication 9.

17. Procédé d'extraction pour la production d'une protéine selon l'une quelconque des revendications 1 à 6, à partir d'une matière organique en contenant, qui comprend la soumission de la matière organique à une macération et à une extraction par solvant.

18. Procédé d'extraction selon la revendication 17, dans lequel la protéine est ensuite purifiée par centrifugation, chromatographie et dialyse.

19. Procédé d'extraction selon la revendication 17, dans lequel la matière organique comprend une graine choisie dans le groupe constitué par la graine de *Capsicum,* la graine de *Briza,* la graine de *Catapodium,* la graine de *Baptisia,* la graine de *Microsensis,* la graine de *Delphinium.*

20. Procédé pour la production d'une protéine selon l'une quelconque des revendications 1 à 6, qui comprend la synthèse chimique de la protéine.
